## Europäisches Patentamt

## European Patent Office

## Office européen des brevets

(11) Publication number: **0 114 442**
**B1**

(12) **EUROPEAN PATENT SPECIFICATION**

(45) Date of publication of patent specification: **07.10.87**

(51) Int. Cl.⁴: **G 01 N 25/14,** C 07 C 126/02, C 07 D 251/60

(21) Application number: **83201834.5**

(22) Date of filing: **22.12.83**

(54) **Process for determining and controlling the composition of aqueous solutions of NH3 and CO2.**

(30) Priority: **24.12.82 NL 8204979**

(43) Date of publication of application:
**01.08.84 Bulletin 84/31**

(45) Publication of the grant of the patent:
**07.10.87 Bulletin 87/41**

(84) Designated Contracting States:
**AT BE DE FR GB IT NL**

(56) References cited:
**US-A-3 940 440**
**US-A-3 957 868**

(73) Proprietor: **STAMICARBON B.V.**
**Mijnweg 1**
**NL-6167 AC Geleen (NL)**

(72) Inventor: **Biermans, Andreas Johannes**
**Slagboomweg 23**
**NL-6129 GA Urmond (NL)**
Inventor: **Burks, Henk Christiaan**
**Drossaertweide 20**
**NL-6438 HV Oirsbeek (NL)**

(74) Representative: **Hoogstraten, Willem Cornelis**
**Roeland et al**
**OCTROOIBUREAU DSM Postbus 9**
**NL-6160 MA Geleen (NL)**

Courier Press, Leamington Spa, England.

Description

The invention relates to a process for determining the composition of aqueous solutions of $NH_3$ and $CO_2$ from which ammonium carbamate or ammonium carbonate 1 aq crystallizes out during cooling to below the saturation temperature. In the following description, reference will be made only to ammonium carbamate but it is to be understood that the statements also apply to ammonium carbonate.

Such solutions occur in practice as process streams in the preparation of urea and in the preparation of melamine. For optimum conversion in the synthesis zone it is necessary, in the preparation of urea, that the water content of carbamate solutions to be recirculated to this zone should be kept as low as permissible with a view to preventing carbamate from crystallizing out. To this end the composition of such a process stream must be known. So far various methods of analysis have been used to analyze the said solutions.

However, in applying these methods the difficulty was that the method of analysis used was too slow for corrective measures to be taken in time in the event of deviations from the correct composition. In US patent specification 3.940.440 it is suggested that various methods has been used for a more or less continuous determination of the carbamate solution concentration. These methods were based upon the monitoring of e.g. density, viscosity, electrical conductivity or specific velocity of sound. In said publication it is proposed to measure the refractive index of the carbamate solution as an indication of its composition. These methods fail to give a correct indication of the composition in that there is not always a reliable relationship between the measured physical property of the solution and its composition. The object of the invention is to provide a process for determing the composition of the said solutions, in which process these difficulties are avoided.

It has now been found that within the range of composition relevant to the practical applications indicated the composition of an aqueous solution of $NH_3$ and $CO_2$ is fully determined if both the density and the saturation temperature thereof are known. If, on the basis of experimental data obtained in respect of solutions having known compositions, a set of saturation isotherms (lines of constant saturation temperature) and a set of lines of constant density are now plotted in, for instance, a triangular diagram $NH_3$—$CO_2$—$H_2O$, it will be found that the lines of the two sets intersect, and by interpolation between these two sets of lines it will be possible to find the composition going with a certain combination of density and saturation temperature.

The process according to the invention is therefore characterized in that the density and the saturation temperature of the solution are measured, the density and saturation temperature of a number of solutions of known compositions are determined, the measured density and saturation temperature are compared with the densities and saturation temperatures of the known compositions, and the composition of the solution is determined from this comparison.

The density depends to some extent on the temperature and must, consequently, be measured at a certain standard temperature, for instance 100°C. If so desired, a density measured at a deviating temperature can be corrected according to the standard temperature.

Equipment for determining the densities of liquids, such as the said solutions, is commercially available. Very suitable are, for instance, vibration-type densimeters, in the application of which the liquid examined is passed through a tube, which is vibrated; the frequency of vibration depends on the density of the liquid to be determined.

If the crystalline phase is ammonium carbamate, preference is given, for determining the saturation temperature, to the use of a process and device as described in the non-prepublished Netherlands patent application 8203013, filed on 28th July 1982, in the name of applicant. (EP—A—0102 106 corresponds to said application, it was published on 07,03,84). In that process the temperature of the solution is gradually raised in an optical measuring vessel from a temperature at which the solution contains crystals of the dissolved substance to a temperature at which all crystals are dissolved, while the temperature is being constantly measured, and a beam of light is passed through the measuring vessel and the dissolution of the last few crystals present is detected optically. The beam of light is a plane-polarized beam of light and the transmitted beam of light is passed through an analyser the polarization direction of which is perpendicular to that of the light beam. The intensity of the light passing through the analyser is measured with a photometer, and the said temperature is determined at the moment when this intensity has reached a low, substantially constant value. For the generation of the plane-polarized light beam preference is given to using a laser. In a measuring cycle a solution not containing any crystals is then fed to the measuring vessel, the temperature of the measuring vessel is the gradually lowered until crystals are formed and the temperature of the measuring vessel is subsequently gradually raised until the crystals dissolve.

Owing to the use of polarized light, the detection of the formation or disappearance of crystals responds exlusively to optically active crystals, such as ammonium carbamate crystals. Optically non-active particles, which solid impurities, if any, usually consist of, therefore do not or hardly disturb the measurement.

If the phase crystallizing out is ammonium carbonate 1 aq, this special method cannot be used, because ammonium carbonate 1 aq crystals are cubic and consequently not optically active; in that case one of the known other methods must be followed to measure the saturation temperature.

The invention also relates to a process for controlling the composition of an aqueous solution of $NH_3$ and $CO_2$ from which ammonium

carbamate or ammonium carbonate 1 aq crystallizes out during cooling to below the saturation temperature. This process is characterized in that the process for measuring the density and the saturation temperature of the solution as described hereinbefore is used and in that the $H_2O$ content is controlled by the supply of water or of an aqueous solution.

Such control can be applied to advantage in the processing of ammonium carbamate solutions or ammonium carbonate solutions formed in the preparation of urea from $NH_3$ and $CO_3$ and in the preparation of melamine from urea.

In the preparation of urea preference is then given to proceeding as follows. After removal of the larger part of the ammonium-carbamate not converted into urea from the synthesis solution formed in the urea synthesis zone, the remaining urea solution still containing ammonium carbamate is separated, in a low-pressure zone, into an aqueous urea solution and a gas mixture containing $NH_3$, $CO_2$ and $H_2O$. This gas mixture is condensed, and the ammonium carbamate solution obtained in this process is recirculated to the urea synthesis zone while supplying an aqueous solution obtained by condensing the gas mixtures separated off in the concentration of the aqueous urea solution. The quantity of aqueous solution supplied is controlled on the basis of the composition of the ammonium carbamate solution to be recirculated to the synthesis zone. In this process the ratio of the percentages on $NH_3$ and $CO_2$ determined on the basis of the saturation temperature and density of the solution can be controlled if so desired by addition of $NH_3$ at the value desired for condensation under optimum conditions.

In the preparation of melamine from urea a gas mixture is formed, in addition to melamine, consisting mainly of $NH_3$ and $CO_2$, from which gas mixture the melamine is separated off. In one such process known in the art an aqueous solution is formed containing the $NH_3$ and $CO_2$ in at least partly bonded form as ammonium carbamate or ammonium carbonate. This solution is usually passed to a urea synthesis plant after the water content has been reduced to a value at which it is certain that crystallization of ammonium carbamate or ammonium carbonate is avoided. In the regulation of this water content, too, the process according to the invention can be applied to advantage.

The invention is elucidated with reference to the drawing. The figures represent the following.

Fig. 1: a triangular diagram $NH_3$—$CO_2$—$H_2O$ with saturation isotherms and lines of constant density indicated therein;

Fig. 2: a highly simplified diagram of part of a urea plant indication how by means of the process according to the invention the composition of a certian process stream can be monitored and controlled.

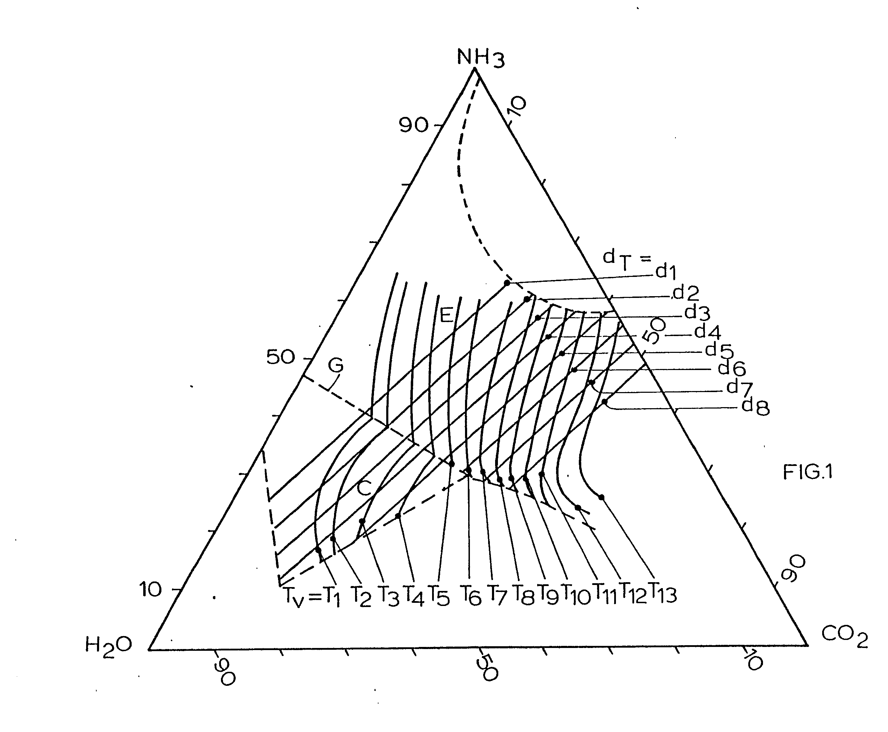

In Fig. 1 the coordinates of the triangular diagram show the composition in % by weight of a solution containing $NH_3$, $CO_2$ and $H_2O$. In the diagram lines of constant saturation temperature $T_v=T_i$ up to and including $T_{13}$ (saturation isotherms) and lines of constant density at a standard temperature of $T$ °C, $d_T=d_1$ up to and including $d_8$ are indicated; the families of lines $T_v$ and $d_T$ are a diagrammatical representation of lines determined experimentally in respect of solutions of known composition. In the diagram E indicates the composition range in which ammonium carbamate crystallizes out during cooling and C the range in which ammonium carbonate 1 aq crystallizes out. The dashed line G indicates the boundary line between ranges E and C. If the values of $T_v$ and $d_T$ of an aqueous solution of $NH_3$ and $CO_2$ have been measured, the composition can be determined by interpolation in the diagram. The values determined by experiment can be stored also in the memory of a microprocessor, and the processor can be programmed with an algorithm which calculates the composition of an unknown solution from its measured values $T_v$ and $d_t$, using the values in the memory.

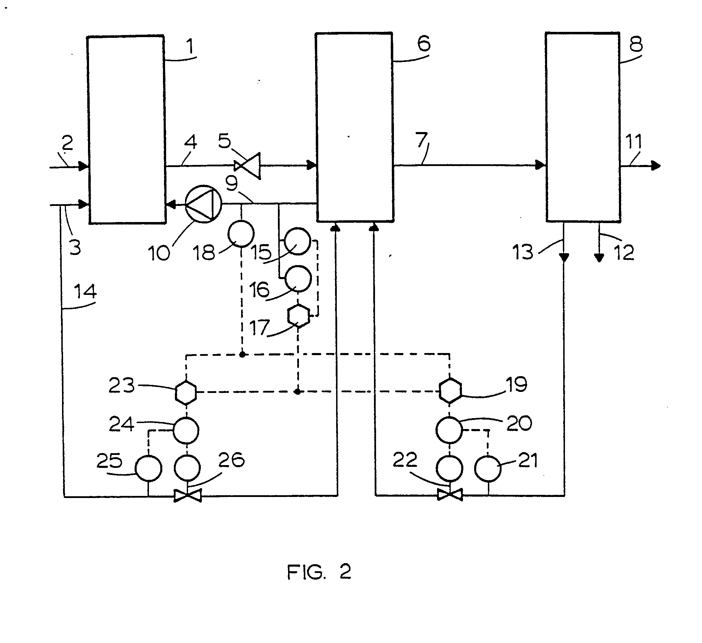

Fig. 2 represents a highly simplified block diagram of a urea plant in which the process according to the invention is applied, by way of non-restrictive example.

Block 1 represents the high-pressure synthesis section of the plant, to which $CO_2$ is supplied through line 2 and $NH_3$ through line 3. In synthesis section 1 a urea synthesis solution is obtained, containing, in addition to urea and water, non-converted $CO_2$ and $NH_3$, partly in bonded form as ammonium carbamate. This synthesis solution is fed through line 4 via reducer 5, in which the pressure of the solution is lowered from, for instance, 10—20 MPa to, for instance, 0.2—2 MPa, to the low-pressure section of the plant represented by block 6. On the one side an aqueous urea solution is obtained herein from the said synthesis solution, which urea solution is supplied through line 7 to the final processing section of the plant represented by block 8, on the other an aqueous solution of $CO_2$ and $NH_3$ (substantially bonded in the form of ammouum carbamate) is obtained, which solution is recirculated through line 9 to the high-pressure synthesis section 1 by means of pump 10, with which the recirculated solution is brought to the high synthesis pressure again.

In the final processing section 8, the urea is separated from water by evaporation of the solution supplied through line 7, usually granulated and carried off at 11 as finished product. The water separated from the urea is largely condensed. Part of this process condensate is carried off from the process through line 12, another part is recirculated through line 13 to low-pressure section 6 to lower the concentration of the ammonium carbamate solution recirculated through line 9 to sythesis section 1. With out this measure the said solution would often be so concentrated that, for instance, in line 9 or pump 10 cystallization might occur, which is undesirable in view of the danger of clogging. However, the amount of process condenstate recirculated through line 13 must not exceed what is required for the certain prevention of this crystallization, because the addition of water will reduce the synthesis efficiency in section 1 and all the water added must, moreover, be evaporated again.

Furthermore, extra $NH_3$ is preferably supplied to low-pressure section 6 through line 14 in order to bring the $NH_3/CO_2$ ratio of the solution recirculated through line 9 to the desired value.

For regulating the quantity of process condensate recirculated through line 13 and the quantity of $NH_3$ supplied through line 14 the process according to the invention is used as follows.

The density of the solution recirculated through line 9 is measured continuously with a densimeter 15, the saturation temperature with a saturation-temperature meter 16. The measuring signals of meters 15 and 16 are passed to a digital signal processing unit 17 generating signals corresponding with the $NH_3$ and $CO_2$ content of the recirculated solution. A flow meter 18 measures the quantity of recirculated solution.

A digital ratio controller 19 receives the measuring signal of flow meter 18 and the analytical signals of the signal processing unit 17 and generates a setting signal for the set-point of flow controller 20, which controls the quantity of process condensate recirculated through line 13. Flow controller 20 receives the measuring signal from a flow meter 21 and controls a control valve 22 in line 13. The quantity of process condensate recirculated through line 13 is set so that the water concentration of the solution recirculated through line 9 to high-pressure section 1 is as low as possible, but not lower than compatible with the certain prevention of the risk of crystallization. To this end a certain margin must be maintained between the temperature of the solution transported through line 9 and the saturation temperature thereof; a suitable value for it is about 5°C when applying the process according to the invention. This is substantially more favourable than that permissible when the quantity of process condensate to be recirculated is determined by means of discontinuous sampling and laboratory analysis of the liquid flowing through line 9, which serve to determine the saturation temperature to be expected. This determination of the crystallization temperature by a roundabout way is, like other indirect methods, relatively inaccurate. In order to prevent crystallization with certainty, the process condensate to be recirculated must therefore be such that the saturation temperature of the solution is about 20°C lower than the temperature prevailing in line 9. In applying the process according to the invention the saturation temperature itself is measured direct and the temperature margin can be much smaller, so that a substantially smaller quantity of water is returned to the synthesis section.

The control of the quantity of $NH_3$ supplied through line 14 is quite analogous to the control of the quantity of recirculated process condensate. Another digital ratio controller 23 also receives the measuring signal from flow meter 18 and the analytical signals of the signal-processing unit 17 and generates a setting signal for the set-point of flow controller 24 controlling the quantity of $NH_3$ supplied through line 14. Flow controller 24 receives the measuring signal from a flow meter 25 and controls a control valve 26 in line 14. The quantity of $NH_3$ supplied through line 14 is set so that the $NH_3/CO_2$ molar ratio of the solution recirculated through line 9 to high-pressure section 1 has a certain optimum value for the process operation in high-pressure section 1, which value is usually between 2.0 and 2.2. The addition of $NH_3$ through line 14 is much smaller than the main supply through line 14, and that usually not more than 2% thereof. In certain cases the process operation may be such that this addition can be omitted.

## Claims

1. Process of determining the composition of aqueous solutions of $NH_3$ and $CO_2$ from which ammonium carbamate or ammonium carbonate 1 aq crystallizes out during cooling to below the saturation temperature, characterized in that the density and the saturation temperature of the solution are measured, the density and saturation temperature of a number of solutions of known compositions are determined, the measured density and saturation temperature are compared with the densities and the saturation temperatures of the known compositions, and the composition of the solution is determined from this comparison.

2. Process of controlling the composition of an aqueous solution of $NH_3$ and $CO_2$ from which ammonium carbamate or ammonium carbonate aq crystallizes out during cooling to below the saturation temperature, characterized in that the composition of the solution is determined by means of the process according to claim 1, and the $H_2O$ content is controlled by the supply of water or of an aqueous solution.

3. Process according to claim 2, characterized in that the solution has been obtained in the preparation of urea from $NH_3$ and $CO_2$.

4. Process according to claim 3, characterized in that the solution has been obtained by separating, in a low-pressure zone, the aqueous urea solution formed in the urea synthesis zone into an aqueous urea solution and a gas mixture containing $NH_3$, $CO_2$ and $H_2O$ after removal of most of the ammonium carbamate not converted into urea and condensing this gas mixture and the ammonium carbamate solution thus obtained is recirculated to the urea synthesis zone while supplying an aqueous solution obtained by condensing the gas mixtures separated off in the concentration of the aqueous urea solution, the quantity of aqueous solution supplied being controlled on the basis of the compositon of the ammonium carbamate solution to be recirculated to the synthesis zone.

5. Process according to claim 4, characterized in that in the condensation of the gas mixture

containing NH$_3$, CO$_2$ and H$_2$O the ratio of the percentages of NH$_3$ and CO$_2$ determined on the basis of the saturation temperature and the density of the ammonium carbamate solution formed is controlled, in the low-pressure zone, at the desired value by addition of NH$_3$.

6. Process according to claim 2, characterized in that the solution has been obtained by absorption, in an aqueous liquid, of NH$_3$ and CO$_2$ released in the preparation of melamine from urea.

**Patentansprüche**

1. Verfahren zur Bestimmung der Zusammensetzung von wässerigen Lösungen von NH$_3$ und CO$_2$ aus denen Ammoniumcarbamat oder Ammoniumcarbonat 1 aq beim Abkühlen uter die Sättigungstemperatur auskristallisiert, dadurch gekennzeichnet, daß die Dichte und die Sättigungstemperatur der Lösung gemessen werden, die Dichte und Sättigungstemperatur einer Anzahl von Lösungen bekannter Zusammensetzungen bestimmt werden, die gemessenen Dichten und Sättigungstemperaturen der bekannten Zusammensetzungen verglichen werden, und die Zusammensetzung der Lösung aus diesem Vergleich bestimmt wird.

2. Verfahren zur Kontrolle der Zusammensetzung einer Wässerigen Lösung von NH$_3$ und CO$_2$, aus denen Ammoniumcarbamat oder Ammoniumcarbonat 1 aq beim Abkülen unter die Sättigungstemperatur auskristallisiert, dadurch gekennzeichnet, daß die zusammensetzung der Lösung mittels des Verfahrens gemäß Anspruch 1 bestimmt und der H$_2$O—Gehalt durch Zufuhr von Wasser oder einer wässerigen Lösung kontrolliert wird.

3. Verfahren nach Anspruch 2, dadurch gekennzeichnet, daß die Lösung aus NH$_3$ und CO$_2$ bei der Herstellung von Harnstoff erhalten wurde.

4. Verfahren nach Anspruch 3, dadurch gekennzeichnet, daß die Lösung durch Trennung, in einer Niederdruckzone, der wässerigen, in einer Harnstoffsynthese gebildeten Harnstofflösung in eine wässerige Harnstofflösung und ein Gasgemisch, das NH$_3$, CO$_2$ und H$_2$O enthält, nach Entfernung des meisten, nicht in Harnstoff umgewandelten Ammoniumcarbamats erhalten wurde, dieses Gasgemisch kondensiert wird, und die so erhaltene Ammoniumcarbamatlösung in die Harstoffsynthese rückgeführt wird, während eine durch Kondensation des bei der Konzentration der wässerigen Harnstofflösung abgetrennten Gasgemisches erhaltene wässerige Lösung zugeführt wird, wobei die Menge an zugeführter wässeriger Lösung auf der Basis der Zusammensetzung der in die Synthesezone rückzuführenden Ammoniumcarbamatlösung kontrolliert wird.

5. Vefahren nach Anspruch 4, dadurch gekennzeichnet, daß bei der Kondensation des NH$_3$, CO$_2$ und H$_2$O enthaltenden Gasgemisches das Verhältnis der Prozentanteile an NH$_3$ und CO$_2$, bestimmt auf der Basis der Sättigungstemperatur und der Dichte der gebildeten Ammoniumcarbamatlösung, in der Niederdruckzone auf dem gewünschten Wert durch Zusatz von NH$_3$ gehalten wird.

6. Verfahren nach Anspruch 2, dadurch gekennzeichnet, daß die Lösung durch Absorption in einer wässerigen Flüssigkeit von NH$_3$ und CO$_2$, freigesetzt bei der Herstellung von Melamin aus Harnstoff, erhalten wurde.

**Revendications**

1. Procédé de détermination de la composition de solutions aqueuses de NH$_3$ et CO$_2$ à partir desquelles le carbamate d'ammonium ou le carbonate d'ammonium I aq cristallise pendant le refroidissement au dessous de la température de saturation, caractérisé en ce qu'on mesure la densité et la température de saturation de la solution, on détermine la densité et la température de saturation d'un certain nombre de solutions de compositions connues, on compare la densisté et la température de saturation mesurées avec les densités et les températures de saturation des compositions connues et on détermine la composition de la solution à partir de cette comparaison.

2. Procédé de réglage de la composition d'une solution aqueuse de NH$_3$ et CO$_2$ à partir de laquelle le carbamate d'ammonium ou le carbonate d'ammonium aq cristallise pendant le refroidissement au dessous de la température de saturation, caractérisé en ce qu'on détermine la composition de la solution par le procédé selon la revendication 1 et on règle la teneur en H$_2$O par l'admission d'eau ou d'une solution aqueuse.

3. Procédé selon la revendication 2, caractérisé en ce qu'on obtient la solution lors de la préparation d'urée à partir de NH$_3$ et CO$_2$.

4. Procédé selon la revendication 3, caractérisé en ce qu'on obtient la solution en séparant dans une zone de basse pression la solution aqueuse d'urée formée dans la zone de synthèse d'urée en une solution aqueuse d'urée et un mélange gazeux contenant NH$_3$, CO$_2$ et H$_2$O après l'évacuation de la majeure partie du carbamate d'ammonium non converti en urée et la condensation de ce mélange gazeux et on remet en circulation la solution ainsi obtenue de carbamate d'ammonium vers la zone de synthèse d'urée tout en fournissant une solution aqueuse qu'on obtient en condensant les mélanges gazeux séparés lors de la concentration de la solution aqueuse d'urée, la quantité de solution aqueuse fournie étant réglée sur la base de la composition de la solution de carbamate d'ammonium qu'on doit remettre en circulation vers la zone de synthèse.

5. Procédé selon la revendication 4, caractérisé en ce qu'on règle la condensation du mélange gazeux contenant HN$_3$, CO$_2$ et H$_2$O, le rapport des pourcentages de NH$_3$ et CO$_2$ déterminés sur la base de la température de saturation et la densité de la solution de carbamate d'ammonium formé, dans la zone basse pression à la valeur désirée par addition de NH$_3$.

6. Procédé selon la revendication 2, caractérisé en ce qu'on obtient la solution par absorption dans un liquide aqueux de NH$_3$ et CO$_2$ dégagés lors de la préparation de la mélamine à partir d'urée.

FIG.1

FIG. 2